# FASCICULE DE BREVET EUROPEEN

(11) **EP 1 230 244 B1**
(45) Date de publication et mention de la délivrance du brevet: **19.01.2005**
(21) Numéro de dépôt: 00974629.8
(22) Date de dépôt: 02.11.2000
(51) Int. Cl.: C07D 495/04, A61K 31/415, C07D 231/56, C07D 231/54

(54) **DERIVES TRICYCLIQUES D'ACIDE PYRAZOLECARBOXYLIQUE, LEUR PREPARATION, LES COMPOSITIONS PHARMACEUTIQUES EN CONTENANT**
TRICYCLISCHE DERIVATE DER PYRAZOLCARBONSÄURE, IHRE HERSTELLUNG UND SIE ENTHALTENDE PHARMAZEUTISCHE ZUBEREITUNGEN
PYRAZOLECARBOXYLIC ACID TRICYCLIC DERIVATIVES, PREPARATION AND PHARMACEUTICAL COMPOSITIONS CONTAINING SAME

(30) Priorité: 03.11.1999 FR 9913846
(43) Date de publication de la demande: 14.08.2002
(73) Titulaire: Sanofi-Aventis, 75013 Paris (FR)
(72) Inventeur: BARTH, Francis, F-34680 Saint-Georges-d'Orques (FR); CONGY, Christian, F-34980 Saint-Gely-du-Fesc (FR); MARTINEZ, Serge, F-34000 Montpellier (FR); RINALDI, Murielle, F-34680 Saint-Georges-d'Orques (FR)
(74) Mandataire: Thouret-Lemaitre, Elisabeth
(86) Numéro de dépôt international: PCT/FR2000/003049
(87) Numéro de publication internationale: WO 2001/032663

(56) Documents cités:
- EP-A- 0 477 049
- EP-A- 0 568 546
- EP-A- 0 576 357
- WO-A-96/09304
- WO-A-97/19063
- US-A- 3 940 418
- HAMILTON R W: "The antiarrhythmic and antiinflammatory activity of a series of tricyclic pyrazoles" JOURNAL OF HETEROCYCLIC CHEMISTRY,US,PROVO, UT, vol. 13, no. 3, 1 juin 1976 (1976-06-01), pages 545-553, XP002085959 ISSN: 0022-152X
- FRAVOLINI, A. ET AL: "Synthesis and pharmacological activity of benzothiopyranopyrazole and benzothiopyranoisoxazole carboxamides" FARMACO, ED. SCI. (1978), 33(11), 855-65, XP002146285

## Description

La présente invention a pour objet des composés antagonistes des récepteurs aux cannabinoïdes CB₁, leur préparation, les compositions pharmaceutiques en contenant. Les composés de l'invention sont des dérivés tricycliques d'acide pyrazolecarboxylique.

Les demandes de brevet EP-A-576 357, EP-A-658 546 et WO-97/19063 décrivent des dérivés du pyrazole présentant une affinité pour les récepteurs aux cannabinoïdes. Plus particulièrement, la demande de brevet EP-A-656 354 revendique le N-pipéridino-5-(4-chlorophényl)-1-(2,4-dichlorophényl)-4-méthylpyrazole-3-carboxamide et ses sels pharmaceutiquement acceptables qui présentent une très bonne affinité pour les récepteurs aux cannabinoïdes CB₁.

La demande de brevet internationale WO-96/09304 décrit des composés inhibant la cyclooxygénase, plus spécifiquement la cyclooxygénase-2. Ces composés utiles dans le traitement de l'inflammation et des maladies inflammatoires répondent à la formule : 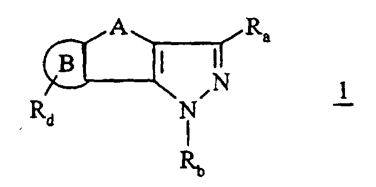 dans laquelle :
A, B, Rₐ, R_{b}, R_{d} ont différentes significations.

On a maintenant trouvé des nouveaux dérivés tricycliques d'acide pyrazolecarboxylique qui possèdent une très bonne affinité pour les récepteurs CB₁ des cannabinoïdes et sont utiles dans les domaines thérapeutiques où le cannabis est connu pour intervenir.

Le Δ⁹-THC est le principal constituant actif extrait de *Cannabis sativa* (Tuner, 1985 ; In Marijuana 84, Ed. Harvey, DY, IRL Press, Oxford).

Les effets des cannabinoïdes sont dûs à une intéraction avec des récepteurs spécifiques de haute affinité présents au niveau central (Devane et al., Mol. Pharmacol., 1988, 34, 605-613) et périphérique (Nye et al., Pharmacol. and Experimental Ther., 1985, 234, 784-791 ; Kaminski et al., 1992, Mol. Pharmacol., 42, 736-742 ; Munro et al., Nature, 1993, 365, 61-65).

La caractérisation des récepteurs a été rendue possible par la mise au point de ligands synthétiques des récepteurs aux cannabinoïdes tels que les agonistes WIN 55212-2 (J. Pharmacol. Exp. Ther., 1993, 264, 1352-1363) ou le CP 55,940 (J. Pharmacol. Exp. Ther., 1988, 247, 1046-1051).

La présente invention a pour objet des composés de formule : 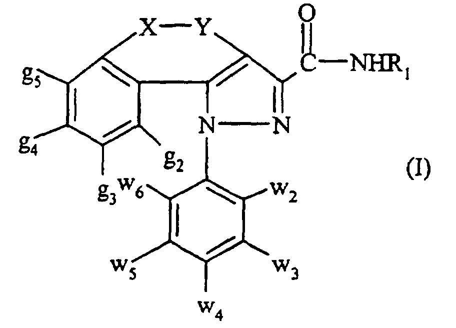 dans laquelle :
- X-Y- représente un groupe choisi parmi -(CH₂)ₙ-CH₂-, -CH₂-S(O)ₚ-, ou -S(O)ₚCH₂- ;
- n est égal à 1 ou 2 ;
- p est égal à zéro, 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₄)alkyle, un (C₁-C₄)alcoxy, un (C₁-C₄)alkylthio, un nitro ; au moins l'un des substituants w₂, w₃, w₄, w₅, w₆ étant différent de l'hydrogène ;
- R₁ représente un radical carbocyclique non aromatique en C₃-C₁₅ non substitué ou substitué une ou plusieurs fois par un (C₁-C₄)alkyle, ou un groupe NR₂R₃ ;
- R₂ et R₃ représentent chacun séparément l'hydrogène ou un (C₁-C₆)alkyle, ou R₂ et R₃ ensemble avec l'atome d'azote auquel ils sont liés constituent un radical hétérocyclique saturé ou insaturé de 5 à 10 chaînons, non substitué ou substitué une ou plusieurs fois par un (C₁-C₄)alkyle ;
ainsi que leurs sels et leurs solvats.

Par alkyle, on entend des alkyles droits ou ramifiés. Les groupes méthyle, éthyle, propyle, isopropyle étant préférés.

Par radical hétérocyclique saturé ou insaturé de 5 à 10 chaînons, on entend un radical hétérocyclique non aromatique mono ou dicyclique, condensé ou ponté, et pouvant comporter un deuxième hétéroarome. Ces radicaux comprennent en particulier les radicaux suivants : 1-pyrrolidinyle, 1-pipéridinyle, 1-hexahydroazépinyle, 4-morpholinyle, 8-azaspiro[4.5]dec-8-yle, bicyclo[2.2.1]heptan-2-yle.

Par radical carbocyclique non aromatique en C₃-C₁₅, on entend un radical saturé, mono ou polycyclique, condensé, ponté ou spiranique ; un radical saturé, condensé ou ponté étant préféré. Ces radicaux comprennent en particulier les radicaux suivants : cyclopentyle, cyclohexyle ou adamantyle ou fenchyle.

Par halogène, on entend un atome de chlore, de brome, de fluor ou d'iode.

Les sels éventuels des composés de formule (I) comprennent les sels d'addition d'acide pharmaceutiquement acceptables tels que le chlorhydrate, le bromhydrate, le sulfate, l'hydrogénosulfate, le dihydrogénophosphate, le méthanesulfonate, le méthylsulfate, le maléate, l'oxalate, le fumarate, le naphtalènesulfonate, le glyconate, le gluconate, le citrate, l'iséthionate, le paratoluènesulfonate, le méthylènesulfonate, le benzènesulfonate ou le succinate.

La présente invention a tout parculièrement pour objet des composés de formule : 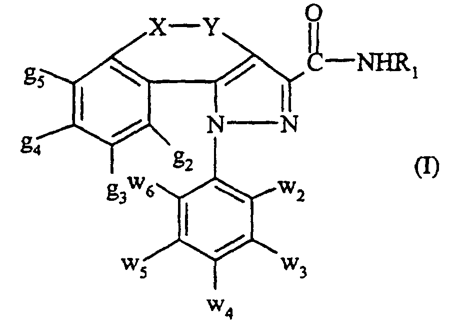 dans laquelle :
- X-Y- représente un groupe choisi parmi -(CH₂)ₙ-CH₂-, -CH₂-S(O)ₚ-, ou -S(O)ₚ-CH₂- ;
- n est égal à 1 ou 2 ;
- p est égal à zéro, 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₃)alkyle, un (C₁-C₃)alcoxy, un (C₁-C₃)alkylthio, un nitro ; au moins l'un des substituants w₂, w₃, w₄, w₅, w₆ étant différent de l'hydrogène ;
- R₁ représente un radical carbocyclique non aromatique en C₃-C₁₅ non substitué ou substitué une ou plusieurs fois par un (C₁-C₄)alkyle, ou un groupe NR₂R₃ ;
- R₂ et R₃ représentent chacun séparément l'hydrogène ou un (C₁-C₆)alkyle, ou R₂ et R₃ ensemble avec l'atome d'azote auquel ils sont liés constituent un radical hétérocyclique saturé ou insaturé de 5 à 10 chaînons, non substitué ou substitué une ou plusieurs fois par un (C₁-C₄)alkyle ;
ainsi que leurs sels et leurs solvats.

Parmi les composés de formule (I), on préfère ceux dans lesquels g₂, g₃, g₅, w₃, w₅, w₆ représentent l'hydrogène et g₄, w₂ et w₄ ont l'une des valeurs définies ci-dessus pour les composés de formule (I), hormis l'hydrogène. Plus particulièrement, on préfère les composés de formule (I) dans lesquels w₂ et w₄ représentent le chlore et g₄ représente le chlore ou le brome.

Parmi les composés de formule (I), on distingue les composés dans lesquels R₁ représente un radical carbocyclique non aromatique en C₃-C₁₅ non substitué ou substitué une ou plusieurs fois par un (C₁-C₄)alkyle.

Parmi les composés de formule (I), on distingue également, les composés dans lesquels R₁ représente NR₂R₃, R₂ et R₃ constituant avec l'atome d'azote auquel ils sont liés un radical hétérocyclique saturé de 5 à 10 chaînons, non substitué ou substitué une ou plusieurs fois par un (C₁-C₄)alkyle.

Parmi les composés de formule (I) on distingue ceux dans lesquels X-Y représente un groupe -(CH₂)ₙ-CH₂-, et ceux dans lesquels X-Y représente un groupe -CH₂-S(O)ₚ- ou un groupe -S(O)ₚ-CH₂-. Les composés de formule (I) dans laquelle X-Y représente -CH₂-CH₂-, -CH₂-CH₂-CH₂- ou -CH₂-S- sont préférés.

Dans la présente description, on utilise les abréviations suivantes :
éther : éther diéthylique
éther iso : éther diisopropylique
EtOH : éthanol
MeOH : méthanol
DCM : dichlorométhane
AcOEt : acétate d'éthyle
LiHMDS : sel de lithium de l'hexaméthyldisilazane
(CO₂Et)₂ : oxalate d'éthyle
APTS : acide paratoluènesulfonique
PPA : acide polyphosphorique
DIBAL : diisobutyl aluminium hydrure
AcOH : acide acétique
TA : température ambiante
F : point de fusion
Eb : point d'ébullition
p : pression
RMN : résonnance magnétique nucléaire. Les spectres RMN sont enregistrés à 200 MHz dans le DMSO d6
s: singulet ; d : doublet ; t : triplet ; m : massif

La présente invention a également pour objet un procédé de préparation d'un composé selon l'invention, de ses sels et de ses solvats. Ce procédé est caractérisé en ce Que l'on traite un dérivé fonctionnel d'un acide de formule : 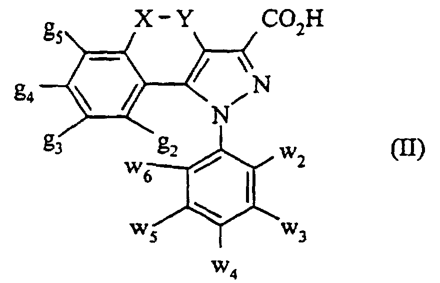 dans laquelle -X-Y- et g₂, g₃, g₄, g₅, w₂, w₃, w₄, w₅, w₆ sont tels que définis ci-dessus pour (I), avec un composé de formule NH₂R₁ (III), dans laquelle R₁ est tel que défini ci-dessus pour (I).

La réaction est effectuée en milieu basique, par exemple en présence de triéthylamine dans un solvant tel que le dichlorométhane ou le tétrahydrofurane.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-N-oxotris(diméthylamino)phosphonium (BOP).

Ainsi par le procédé selon l'invention, on peut faire réagir le chlorure de l'acide de formule (II) obtenu par réaction du chlorure de thionyle sur l'acide de formule (II) dans un solvant inerte tel que le benzène ou le toluène ou un solvant chloré (le dichlorométhane, le dichloroéthane, le chloroforme par exemple), un éther (tétrahydrofurane, dioxane par exemple), ou un amide (N,N-diméthylformamide par exemple) sous un atmosphère inerte, à une température comprise entre 0°C et la température de reflux du solvant.

Une variante au mode opératoire consiste à préprarer l'anhydride mixte de l'acide de formule (II) par réaction du chloroformiate d'éthyle avec l'acide de formule (II), en présence d'une base telle que la triéthylamine.

Quelques acides de formule (II) ont été décrits dans la littérature.

La publication de Fravolini et al., Farmaco ed. Sci., 1978, 33 (11), 855-865 décrit des composés de formule : 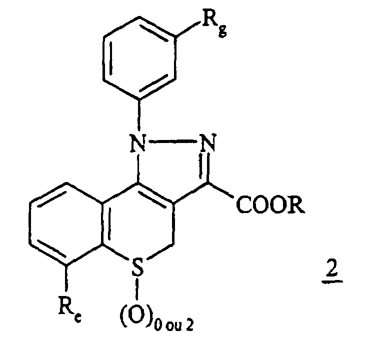 qui sont des intermédiaires pour préparer des composés testés pour des activités antiinflammatoires, analgésiques et cardiovasculaires.

Parmi les composés décrits, on trouve les valeurs suivantes pour les substituants :
Rₑ = H et R_{g} = F ;
Rₑ = H et R_{g} = CF₃.

La publication de Hamilton dans J. Het. Chem., 1976, 13 (3), 545-553 et le brevet US 3 940 418 décrivent des composés de formule :

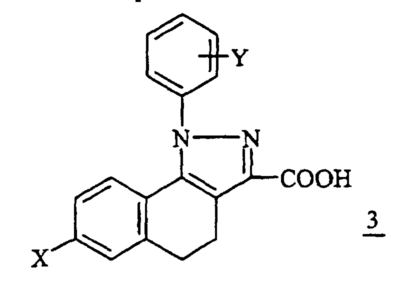

Parmi ces composés, les substituants suivants sont décrits :
Y = p-Cl et X = H ou X = OCH₃.

A part les composés cités ci-dessus, les acides de départ (II) sont nouveaux et constituent un autre aspect de la présente invention, leurs dérivés fonctionnels sont également nouveaux, notamment leurs chlorures d'acide et leurs esters alkyliques en C₁-C₄.

Ainsi la présente invention a également pour objet les acides de formule : 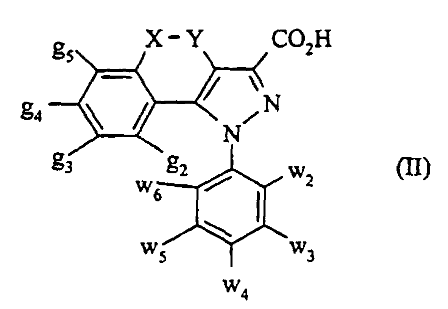 dans laquelle g₂, g₃, g₄, g₅, w₂, w₃, w₄, w₅, w₆ sont tels que définis pour (I) à la revendication 1 et leurs dérivés fonctionnels, à la condition que :
- lorsque X-Y est -S-CH₂ ou -SO₂-CH₂-, et w₅ représente un fluor ou un trifluorométhyle, g₂, g₃, g₄, g₅, w₂, w₃, w₄, w₅, w₆ ne représentent pas simultanément l'hydrogène ;
- lorsque X-Y est -CH₂-CH₂-, w₄ représente un chlore, un fluor ou un méthoxy et g₄ représente l'hydrogène, un chlore ou un méthoxy, g₂, g₃, g₅, w₂, w₃, w₅, w₆ ne représentent pas simultanément l'hydrogène. >
L'acide de formule (II) est obtenu en suivant le schéma réactionnel ci-après :

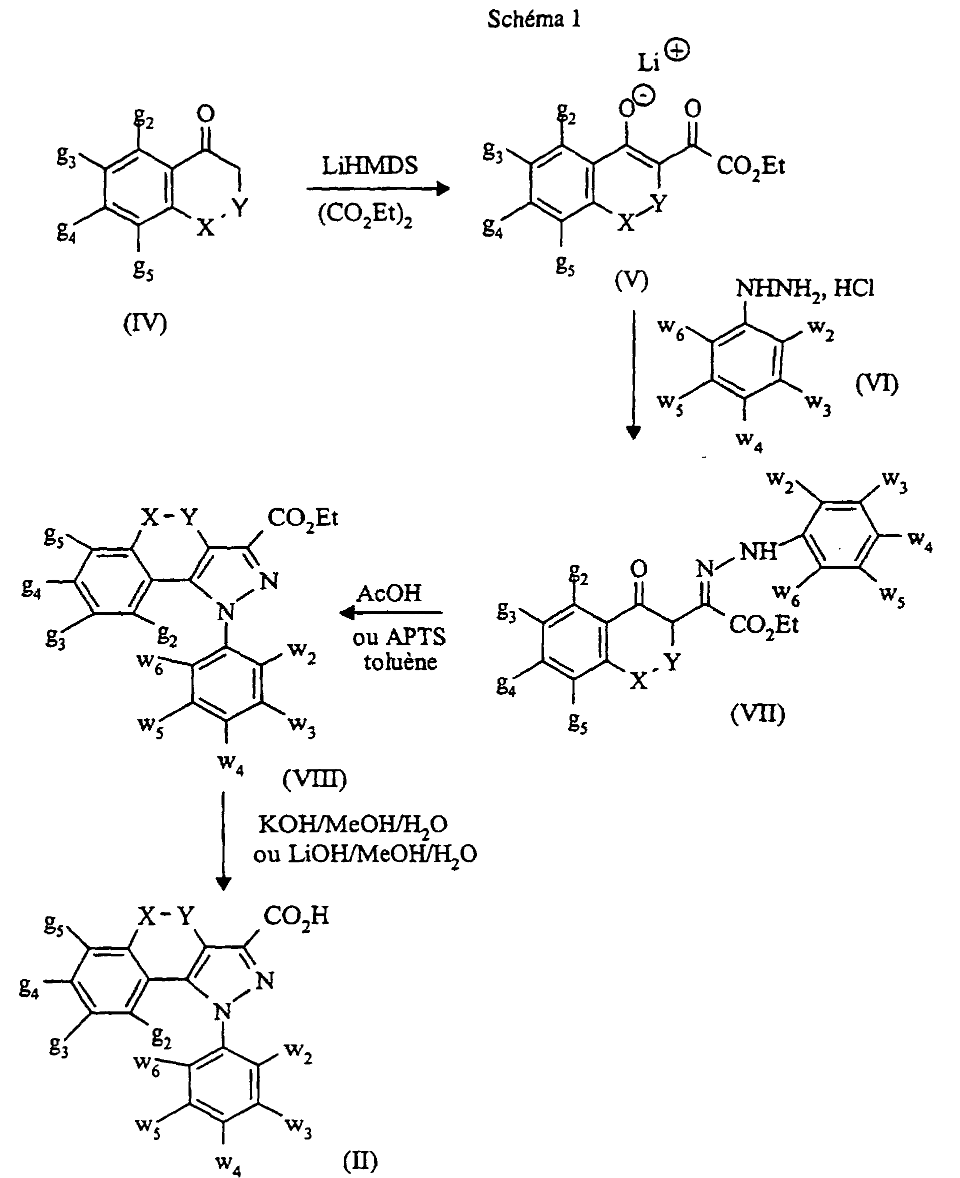

Les thiochroman-4-ones (IV, -X-Y- = -S-CH₂-) et les isothiochroman-4-ones (IV, -X-Y- = -CH₂-S-) sont préparées selon les méthodes décrites dans WO-96/09304. En particulier, la 7-chloroisothiochroman-4-one, la 7-fluoroisothio chroman-4-one ainsi que la 7-chlorothiochromane-4-one sont décrites dans cette demande internationale WO-96/09304.

Lorsque -X-Y- représente respectivement -CH₂-SO-, CH₂-SO₂- ou respectivement -SO-CH₂-, SO₂CH₂-, on prépare un composé de formule (VIII) dans laquelle -X-Y- représente respectivement -CH₂-S- ou respectivement -S-CH₂ et on fait agir un agent oxidant tel que l'acide métachloroperbenzoïque, soit en quantité équimolaire pour obtenir un sulfoxide, soit en quantité double pour obtenir une sulfone.

Les tétralones de formule (IV) dans laquelle -X-Y- représente -CH₂-CH₂- sont connues ou préparées par des méthodes connues telles que décrites dans Synthetic Communications, 1991, 21, 981-987.

Les benzosubérones de formule (IV) dans laquelle -X-Y- représente -(CH₂)₂-CH₂- sont connues ou préparées selon J. Med. Chem., 1991, 37, 3482-3491 et J. Org. Chem., 1962, 27, 7076.

On prépare le sel de lithium de formule (V) par action d'une base lithiée tel que l'hexaméthyldisilazane puis de l'oxalate d'éthyle.

Par action du chlorhydrate de phénylhydrazine (VI), on prépare le composé de formule (VII) ; celui-ci est ensuite cyclisé par chauffage en présence d'acide acétique ou en présence d'acide paratoluènesulfonique dans le toluène. On effectue ensuite une saponification selon les méthodes classiques, par exemple en présence de potasse ou d'hydroxyde de lithium dans le méthanol, pour obtenir l'acide attendu de formule (II).

Les dérivés aminés de départ de formule (III) sont connus ou préparés par des méthodes connues en particulier celles décrites dans EP-0658546. Ainsi la 8-azaspiro[4.5]dec-8-ylamine est préparée à partir du 8-azaspiro[4.5]décane, lui-même préparé selon J. Med. Chem., 1964, 7, 784-786 ou Bull. Soc. Chem. Fr., 1964, 2572-2579. La (1S)-endo-1,3,3-triméthylbicyclo[2.2.1]heptan-2-ylamine est préparée selon J. Am. Chem. Soc., 1951, 73, 3360 ou selon J. Med. Chem., 1991, 34, 1003. La 4,4-diméthyl-pipéridin-1-ylamine est préparée selon J. Med. Pharm. Chem., 1962, 5, 815.

Le composé de formule (I) obtenu par le procédé selon l'invention est isolé, sous forme de base libre ou de sel ou de solvate, selon les techniques conventionnelles.

Le composé de formule (I) peut être isolé sous forme d'un de ses sels, par exemple le chlorhydrate ou l'oxalate ; dans ce cas, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée en un autre sel comme le méthanesulfonate, le fumarate ou le 2-naphtalènesulfonate.

Lorsque le composé de formule (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

Les composés de formule (I) possèdent une très bonne affinité *in vitro* pour les récepteurs aux cannabinoïdes CB₁, dans les conditions expérimentales décrites par Devane et al., Mol. Pharmacol., 1988, 34, 605-613.

Plus particulièrement, les composés de la présente invention, tels quels ou sous forme d'un de leurs sels pharmaceutiquement acceptables, sont des antagonistes puissants et sélectifs des récepteurs aux cannabinoïdes CB₁, ayant un Ki inférieur à 5.10⁻⁷M. Ils sont au moins 10 fois plus actifs sur les récepteurs CB₁ que sur les récepteurs CB₂.

D'autre part, leur nature antagoniste a été démontrée par les résultats dans les modèles de l'inhibition de l'adénylate-cyclase comme décrit dans M. Rinaldi-Carmona et al., J. Pharmacol. Exp. Ther., 1996, 278, 871-878.

La toxicité des composés (I) est compatible avec leur utilisation en tant que médicament.

Selon un autre de ses aspects, la présente invention concerne l'utilisation des composés de formule (I), ou de l'un de leurs sels et solvates pharmaceutiquement acceptables, pour la préparation de médicaments destinés à traiter les maladies impliquant les récepteurs aux cannabinoïdes CB₁.

Par exemple et de manière non limitative, les composés de formule (I) sont utiles comme médicaments psychotropes, notamment pour le traitement des troubles anxieux, des troubles de l'humeur, des troubles délirants, des troubles psychotiques en général, pour le traitement de la schizophrénie, de la dépression, ainsi que pour le traitement des troubles liés à l'utilisation de substances psychotropes, notamment dans le cas d'un abus d'une substance et/ou dépendance à une substance, y compris la dépendance alcoolique.

Les composés (I) selon l'invention peuvent être utilisés comme médicaments pour le traitement des neuropathies, de la migraine, du stress, des maladies d'origine psychosomatique, de l'épilepsie, des troubles du mouvement, en particulier des dyskinésies ou de la maladie de Parkinson.

Les composés (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance et comme neuroprotecteur.

Les composés (I) selon l'invention peuvent être utilisés comme médicaments dans les troubles de l'appétit, de l'appétence (pour les sucres, carbohydrates, drogues, alcools ou toute substance appétissante) et/ou des conduites alimentaires, notamment en tant qu'anorexigène ou pour le traitement de l'obésité ou de la boulimie ainsi que pour le traitement du diabète de type II ou diabète non insulinodépendant et dans le sevrage tabagique. De plus, les composés de formule (I) selon l'invention peuvent être utilisés en tant que médicaments dans les troubles gastro-intestinaux, les vomissements, les troubles vésicaux et urinaires, les troubles cardio-vasculaires, les troubles de la fertilité, les phénomènes inflammatoires, les maladies infectieuses ainsi qu'en tant que médicaments pour la chimiothérapie anticancéreuse.

Les composés selon la présente invention peuvent également être utiles dans le traitement des pathologies neuroinflammatoires, en particulier les maladies entraînant une démyélisation telles que la sclérose en plaques ou le syndrome de Guillain-Barré, ainsi que les encéphalites virales, les accidents vasculaires cérébraux, les traumatismes crâniens, par exemple.

Selon la présente invention, les composés de formule (I) sont tout particulièrement utiles pour le traitement des troubles psychotiques, en particulier la schizophrénie ; pour le traitement des troubles de l'appétit et de l'obésité, pour le sevrage tabagique et pour le traitement des troubles mnésiques et cognitifs.

Selon un de ses aspects, la présente invention est relative à l'utilisation d'un composé de formule (I) pour le traitement des troubles et maladies indiqués ci-dessus.

Selon un autre de ses aspects, la présente invention concerne également l'utilisation des composés de formule (I), tels quels ou sous forme radiomarquée comme outils pharmacologiques chez l'homme ou chez l'animal, pour la détection et le marquage des récepteurs CB₁.

Les composés selon l'invention sont généralement administrés en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvates.

Les composés de formule (I) ci-dessus et leurs sels ou solvates pharmaceutiquement acceptables peuvent être utilisés à des doses journalières de 0,01 à 100 mg par kg de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2 à 1000 mg par jour selon l'âge du sujet à traiter ou le type de traitement, à savoir prophylactique ou curatif.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,1 à 1000 mg, avantageusement de 0,5 à 500 mg, de préférence de 1 à 200 mg dudit principe actif par unité de dosage pour les administrations quotidiennes.

Lorsque l'on prépare une composition solide sous forme de comprimé, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intra-oculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse, on peut utiliser un cosolvant comme par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades ou des gels.

Pour l'administration transdermique, on peut utiliser des patches sous forme multilaminée ou à réservoirs dans lesquels le principe actif peut être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ-cyclodextrine, 2-hydroxypropyl-β-cyclodextrine ou méthyl-β-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

### PREPARATION 1

### Acide 7-chloro-1-(2,4-dichlorophényl)-1,5-dihydroisothiochroméno[4,3-c]-pyrazole-3-carboxylique.

II : g₄ = w₂ = w₄ = Cl, X-Y = CH₂-S.

### A) 7-Chloroisothiochroman-4-one.

Ce composé est préparé selon le mode opératoire décrit dans le demande de brevet internationale WO 96/09304.

### B) Ester éthylique du sel de lithium de l'acide (7-chloro-4-oxido-1H-isothiochromen-3-yl)oxoacétique.

On refroidit à -60°C une solution de 3,44 g de sel de lithium de l'hexaméthyldisilazane dans 85 ml d'éther diéthylique et ajoute, sous atmosphère d'azote et goutte à goutte, une solution de 3,71 g du composé obtenu à l'étape précédente dans 60 ml d'éther diéthylique. On laisse la température du mélange réactionnel remonter à -30°C, ajoute en une seule fois 2,8 ml d'oxalate de diéthyle et laisse 18 heures sous agitation à TA. On essore le précipité formé, le lave à l'éther diéthylique et le sèche sous vide. On obtient 4,58 g du produit attendu qui est utilisé tel quel à l'étape suivante.

### C) Ester éthylique de l'acide (7-chloro-4-oxo-isothiochroman-3-yl)-(2,4-dichloro phénylhydrazono)acétique.

A une suspension de 2,50 g du composé obtenu à l'étape précédente dans 35 ml d'EtOH, on ajoute à TA 1,75 g du chlorhydrate de 2,4-dichlorophénylhydrazine et laisse 4 heures sous agitation à TA. On essore le précipité formé, le lave à l'EtOH et le sèche sous vide pour obtenir un premier jet. On concentre sous vide de moitié les jus d'essorage et de lavage et laisse 16 heures sous agitation à TA. On essore le précipité formé, le lave à l'EtOH et le sèche sous vide pour obtenir un deuxième jet. On obtient au total 1,18 g du produit attendu, F = 170°C.

### D) Ester éthylique de l'acide 7-chloro-1-(2,4-dichlorophényl)-1,5-dihydroisothiochroméno[4,3-c]pyrazole-3-carboxylique.

On chauffe au reflux pendant 18 heures un mélange de 2,09 g du composé obtenu à l'étape précédente et 55 ml d'acide acétique, puis le laisse 56 heures sous agitation à TA. On verse le mélange réactionnel dans 500 ml d'eau glacée, essore le précipité formé, le lave à l'eau et le sèche sous vide. On obtient 1,93 g du produit attendu, F = 95°C.

### E) Acide 7-chloro-1-(2,4-dichlorophényl)-1,5-dihydroisothiochroméno[4,3-c]-pyrazole-3-carboxylique.

A une solution de 0,91 g du composé obtenu à l'étape précédente dans 30 ml de MeOH, on ajoute une solution de 0,31 g de KOH dans 10 ml d'eau, puis chauffe au reflux pendant 3 heures. On verse le mélange réactionnel sur une solution de 10 ml d'H₂SO₄ à 5 % et 300 ml d'eau glacée, extrait au DCM, lave la phase organique à l'eau, par une solution saturée de NaCl, sèche sur MgSO₄ et évapore sous vide le solvant. On obtient 0,7 g du produit attendu, F = 262°C.
RMN : 4,10 ppm : s : 2H ; 6,60 ppm : d : 1H ; 7,25 ppm : d : 1H ;
7,65-8,10 : m : 4H.

### PREPARATION 2

### Acide 7-chloro-1-(2,4-dichlorophényl)-4,4-dioxo-4,5-dihydro-1H-4λ⁶-isothiochromeno[4,3-c]pyrazole-3-carboxylique.

II : g₄ = w₂ = w₄ = Cl, X-Y = CH₂-SO₂.

### A) Ester éthylique de l'acide 7-chloro-1-(2,4-dichlorophényl)-4,4-dioxo-4,5-dihydro-1H-4λ⁶-isothiochromeno[4,3-c]pyrazole-3-carboxylique.

On dissout 2,96 g d'acide metachloroperbenzoïque dans 45 ml de DCM et cette solution est versée goutte à goutte en 1 heure, à une température comprise entre 0°C et 5°C sur une solution contenant 2,51 g de l'ester obtenu à la PREPARATION 1, étape D, en solution dans 80 ml de DCM. Après 3 heures sous agitation à température ambiante, on ajoute 250 ml d'une solution à 10 % de Na₂CO₃ puis on laisse 10 minutes sous agitation. On extrait au DCM, puis on lave par une solution à 10 % de Na₂CO₃, par une solution de NaCl, puis 2 fois par une solution aqueuse saturée de NaCl. On obtient 2,58 g du composé attendu.

### B) Acide 7-chloro-1-(2,4-dichlorophényl)-4,4-dioxo-4,5-dihydro-1H-4λ⁶-isothiochromeno[4,3-c]pyrazole-3-carboxylique

On place 505 mg d'ester obtenu à l'étape précédente en solution dans 15 ml de méthanol et on ajoute 133 mg de LiOH et 1 ml d'eau. Après 2 heures sous agitation à TA, on concentre le méthanol sous vide et on reprend le résidu par 10 ml d'eau glacée. Le mélange est acidifié à pH = 2 par HCl 1N puis on filtre le précipité obtenu, on le lave à l'eau et on le sèche sous vide pour obtenir 0,457 g du composé attendu sous forme d'un solide amorphe.
RMN : 5,10 ppm : système AB : 2H ; 6,80 ppm : d : 1H ; 7,55 ppm : dd : 1H ;
7,80-8,15 ppm : m : 4H.

### PREPARATION 3

### Acide 7-chloro-1-(2,4-dichlorophényl)-4-oxo-4,5-dihydro-1H-4λ⁴-isothiochromeno[4,3-c]pyrazole-3-carboxylique.

II : g₄ = w₂ = w₄ = Cl, X-Y = CH₂-SO.

### A) Ester éthylique de l'acide 7-chlora-1-(2,4-dichlorophényl)-4-oxo-4,5-dihydro-1H-4λ⁴-isothiochromeno[4,3-c]pyrazole-3-carboxylique.

On prépare une solution de 0,86 g d'acide métachloroperbenzoïque dans 100 ml de DCM puis on l'ajoute goutte à goutte à une solution à 0°C contenant 1,47 g d'ester obtenu à la PREPARATION 1, étape D en solution dans 100 ml de DCM. Après 3 heures et demie sous agitation, on ajoute 50 ml de solution à 5 % de Na₂CO₃ puis on agite 15 minutes. On extrait au DCM puis on lave par une solution saturée puis une solution diluée de NaCl. Après évaporation des solvants et séchage, le résidu est chromatographié sur silice en éluant par un mélange toluène/AcOEt (90/10 ; v/v). On obtient 0,99 g du composé attendu.

### B) Acide 7-chloro-1-(2,4-dichlorophényl)-4-oxo-4,5-dihydro-1H-4λ⁴-isothiochromeno[4,3-c]pyrazole-3-carboxylique

A une solution de 960 mg d'ester obtenu à l'étape précédente dans 40 ml de méthanol, on ajoute une solution contenant 0,265 g d'hydroxyde de lithium dans 3 ml d'eau. Après 2 heures sous agitation, on concentre le méthanol à sec, reprend avec le l'eau glacée, puis acidifie le mélange à pH = 2 par addition d'HCl 1N. Le précipité blanc obtenu est filtré, lavé à l'eau puis séché sous vide. On obtient 0,7 g du composé attendu sous forme d'un solide amorphe.
RMN : 5,75-6,00 ppm : m : 1H ; 6,50-6,70 ppm : m : 1H ;
7,20-8,10 ppm : m : 6H.

### PREPARATION 4

### Acide 7-chloro-1-(2,4-dichlorophényl)-4,5-dihydro-1H-benzo[g]indazole-3-carboxylique.

II : g₄ = w₂ = w₄ = Cl, X-Y = -(CH₂)₂-.

### A) 6-Chloro-3,4-dihydro-2H-naphtalen-1-one.

Ce composé est préparé selon Synthetic Commun., 1991, 21 (8 et 9), 981-987.

### B) Ester éthylique du sel de lithium de l'acide (6-chloro-1-oxido-3,4-dihydronaphtalen-2-yl)oxoacétique.

A -60°C, sous azote, on place 6,34 g de sel de lithium de l'hexaméthyldisilazane dans 100 ml d'éther anhydre puis on verse goutte à goutte une solution contenant 6,2 g du composé de l'étape A dans 50 ml d'éther. On maintient le milieu réactionnel à environ 30°C pendant 30 minutes puis on ajoute 5,15 ml d'oxalate d'éthyle. Après une nuit sous agitation, on filtre le précipité obtenu, rince à l'éther et sèche sous vide pour obtenir 8,2 g du composé attendu.

### C) Ester éthylique de l'acide 6-chloro-1-oxo-1,2,3,4-tétrahydronaphtalen-2-yl)-[2,4-dichlorophénylhydrazino]acétique.

On place 5,23 mg du sel de lithium obtenu à l'étape précédente dans 50 ml d'éthanol et on ajoute 3,9 g de chlorhydrate de 2,4-dichlorophénylhydrazine. Après 6 heures à TA sous agitation, on filtre le précipité formé, rince à l'éthanol et sèche sous vide pour obtenir 3,8 g du composé attendu.

### D) Ester éthylique de l'acide 7-chloro-1-(2,4-dichlorophényl)-4,5-dihydro-1H-benzo [g]indazole-3-carboxylique.

On chauffe à reflux pendant 11 heures, un mélange contenant 3,8 g du composé obtenu à l'étape précédente et 50 ml d'acide acétique. On ajoute 20 ml d'eau glacée puis on filtre le précipité obtenu, rince à l'eau glacée et sèche sous vide. Le résidu est chromatographié sur silice en éluant par un mélange cyclohexane/AcOEt (90/10 ; v/v) et l'on obtient 2 g du composé attendu.

### E) Acide 7-chloro-1-(2,4-dichlorophényl)-4,5-dihydro-1H-benzo[g]indazole-3-carboxylique.

On place 2 g d'ester obtenu à l'étape précédente dans 30 ml de MeOH, on ajoute 0,66 g de potasse dans 4,2 ml d'eau puis on chauffe 2 heures à reflux et laisse une nuit à TA.

On concentre sous vide, reprend le résidu par de l'eau glacée, acidifie à pH = 1 par addition d'HCl 1N puis on extrait par AcOEt et lave par une solution saturée de NaCl. On obtient 1,8 g du composé attendu. F = 237°C.
RMN : 2,75-3,00 ppm : m : 4H ; 6,35 ppm : d : 1H ; 7,10 ppm : dd : 1H ;
7,40 : d : 1H ; 7,60-7,75 ppm : m : 2H ; 7,90 : d : 1H.

### PREPARATION 5

### Acide 7-trifluorométhyl-1-(2,4-dichlorophényl)4,5-dihydro-1H-benzo[g] indazole-3-carboxylique.

II : g₄ = CF₃, w₂ = w₄ =Cl, X-Y = -(CH₂)₂-.

### A) (3-trifluorométhyl)phényltributylétain.

On place 5,62 g de magnésium dans 42 ml d'éther et on ajoute 31 ml de (3-trifluorométhyl)bromobenzène puis on chauffe 8 heures à reflux et laisse 1 heure à TA. A cette solution, on ajoute 46,3 ml de chlorure de tributylétain dilué dans 46 ml d'éther. Après 2 heures à TA, on chauffe 6 heures à reflux puis on hydrolyse par addition de 140 ml d'eau en refroidissant à une température comprise entre 0°C et 5°C. Après distillation sous vide, on obtient 65 g du composé attendu.

### B) Ester méthylique de l'acide 4-(3-trifluorométhylphényl)but-2-ènoïque.

Dans un tricol, on introduit 0,765 g de chlorure de bis(triphénylphosphine) palladium (II) en suspension dans 180 ml de THF puis on ajoute goutte à goutte 2,58 ml de DIBAL (1M dans le toluène) et on laisse sous agitation 45 minutes à TA. On ajoute ensuite un mélange contenant 65 g du composé préparé à l'étape précédente et 27 g d'ester méthylique d'acide 4-bromobut-2-enoïque dans 100 ml de THF. Le milieu réactionnel est chauffé à reflux pendant 16 heures puis on filtre sur Célite® et concentre le filtrat à sec. Le résidu est chromatographié sur silice en éluant par un mélange cyclohexane/AcOEt (90/10 ; v/v). On obtient 44 g du composé attendu.

### C) Ester méthylique de l'acide 4-(3-trifluorométhylphényl)butanoïque.

On agite pendant 6 jours sous une pression de 4 bars d'hydrogène 26 g du composé obtenu à l'étape précédente et 1,8 g de Pd/C à 10 % dans 500 ml d'éthanol. Après évaporation des solvants et séchage, le résidu est chromatographié sur silice en éluant par un mélange cyclohexane/AcOEt (90/10 ; v/v). On obtient 15,83 g du composé attendu.

### D) Acide 4-(3-trifluorométhylphényl)butanoïque.

15,83 g d'ester obtenu à l'étape précédente sont dilués dans 150 ml de THF et on ajoute une suspension de 5,37 g de LiOH dans 10,7 ml d'eau. Après une nuit de chauffage à reflux, on concentre le THF puis on reprend le résidu par de l'eau à une température inférieure à 5°C et on acidifie à pH = 2 par HCl 1N. On extrait à l'éther, décante et lave à l'eau pour obtenir 14 g du composé attendu.

### E) 6-(trifluorométhyl)-3,4-dihydro-2H-naphtalen-1-one.

On mélange 9,5 g d'acide obtenu à l'étape précédente et 270 g de PPA et on chauffe à 65°C pendant 2 heures. On ajoute 500 ml d'eau puis, après refroidissement, on extrait par AcOEt et décante. On lave par de l'eau, une solution à 5 % de Na₂CO₃, une solution saturée de NaCl puis on sèche et le résidu est chromatographié sur silice en éluant par un mélange cyclohexane/AcOEt (94/6 ; v/v). On obtient 1,6 g du composé attendu.

### F) Acide 7-trifluorométhyl-1-(2,4-dichlorophényl)4,5-dihydro-1H-benzo[g] indazole-3-carboxylique.

On procède ensuite comme décrit à la PREPARATION 1 pour obtenir le composé attendu. F = 245 °C
RMN : 2,95-3,25 ppm : m : 4H ; 6,75 ppm : d : 1H ; 7,55 ppm : d : 1H ;
7,75-7,95 ppm : m : 3H ; 8,10 : d : 1H.

### PREPARATION 6

### Acide 1-(2,4-dichlorophényl)-1,4,5,6-tétrahydro-1,2-diazabenzo[e]azulène-3-carboxylique.

II : g₄ = H, w₂ = w₄ =Cl, X-Y = -(CH₂)₃-.

La benzosubérone (6,7,8,9-tétrahydrobenzocyclohepten-5-one) est commerciale.

### A) Ester éthylique du sel de lithium de l'acide (5-oxido-8,9-dihydro-7-benzocyclohepten-6-yl)oxoacétique.

Sous azote, on introduit 5,74 g de sel de lithium d'hexaméthyldisilazane en suspension dans 90 ml d'éther et on refroidit à la température de -60°C puis on ajoute en 15 minutes une solution contenant 5 g de benzosubérone dans 60 ml d'éther. On laisse remonter la température à -30°C puis on ajoute rapidement 4,7 ml d'oxalate d'éthyle. Après retour à TA, on laisse sous agitation 4 heures puis on filtre le précipité formé, rince à l'éther et sèche sous vide pour obtenir 8,1 g du composé attendu.

### B) Ester éthylique de l'acide 5-oxo-6,7,8,9-tétrahydro-5H-benzocyclohepten-6-yl)-(2,4-dichlorophénylhydrazono)acétique.

On mélange 8,1 g du sel de lithium obtenu à l'étape précédente dans 100 ml d'éthanol et 6,5 g de chlorhydrate de 2,4-dihydrophénylhydrazine. Après 4 heures sous agitation à TA, on filtre le solide obtenu, rince à l'éthanol et sèche sous vide pour obtenir 5,75 g du composé attendu.

### C) Ester éthylique de l'acide 1-(2,4-dichlorophényl)-1,4,5,6-tétrahydro-1,2-diazobenzo[e]azulène-3-carboxylique.

On chauffe à reflux pendant 6 heures un mélange contenant 5,75 g du composé obtenu à l'étape précédente et 60 ml d'acide acétique. Après refroidissement, on verse le milieu réactionnel sur de l'eau glacée. On filtre le produit formé, lave à l'eau et sèche sous vide pour obtenir 5,07 g du composé attendu.

### D) Acide 1-(2,4-dichlorophényl)-1,4,5,6-tétrahydro-1,2-diazobenzo[e]azulène-3-carboxylique.

On place 5 g de l'ester obtenu à l'étape précédente dans 75 ml de méthanol et on ajoute 1,8 g de potasse dans 20,8 ml d'eau puis on chauffe à reflux pendant 1 heure. On concentre sous vide, reprend le résidu dans l'eau glacée, acidifie à pH = 1 par addition d'HCl 1N puis on filtre le produit attendu. On obtient 4,5 g. F = 262°C.
RMN : 2,10-2,35 ppm : m : 2H ; 2,60-3,25 ppm : m : 6H ; 6,75 ppm : d : 1H ;
7,10-7,45 ppm : m : 3H ; 7,70 ppm : dd : 1H ; 7,80-7,90 ppm : m : 2H.

### PREPARATION 7

### Acide 8-chloro-1-(2,4-dichlorophényl)-1,4,5,6-tétrahydro-1,2-diazabenzo[e] azulène-3-carboxylique.

II : g₄ = w₂ = w₄ = CI, X-Y = (CH₂)₃-.

### A) Acide 5-(3-(N-acétyl)aminophényl)pentanoïque.

Ce composé est préparé selon N.L. Allinger et al., (J. Org. Chem. 1962, 27, 70-76.

### B) 2-(N-acétyl)amino-6,7,8,9-tétrahydrobenzocyclohepten-5-one.

3,75 g d'acide décrit à l'étape précédente dans 20 ml de DCM, sont ajoutés, sous forte agitation, à 130 g d'acide phosphorique. On chauffe pendant 3 heures à 100°C puis on refroidit à l'aide d'un bain de glace et ajoute lentement 200 ml d'eau, puis on extrait à l'acétate d'éthyle et décante. La phase organique est lavée à l'eau, par une solution aqueuse de Na₂CO₃ à 5 % puis par une solution saturée de NaCl. On obtient une huile qui concrétise dans l'éther isorpropylique pour donner 2,24 g du composé attendu, F = 105°C.

### C) Chlorhydrate de 2-amino-6,7,8,9-tétrahydrobenzocyclohepten-5-one.

On place 2,6 g du dérivé N-acétylé obtenu à l'étape précédente en suspension dans une solution d'HCl 6N et on chauffe à reflux pendant 2 heures et demie. La solution obtenue est utilisée telle quelle à l'étape suivante.

### D) 2-Chloro-6,7,8,9-tétrahydrobenzocyclohepten-5-one.

La solution obtenue à l'étape précédente est refroidie entre 0°C et 5°C et on ajoute goutte à goutte une solution de 1,02 g de nitrite de sodium dans 5 ml d'eau puis on maintient 15 minutes sous agitation. La solution de diazoninium ainsi formée et coulée sur une solution de 1,22 g de CuCl dans 6 ml d'HCl 6N maintenue à une température comprise entre 0°C et 5°C. Après 1 heure à 5°C, on laisse revenir à TA puis on ajoute 15 ml d'eau et extrait par un mélange éther-acétate d'éthyle (80/20 ; v/v) puis on concentre sous vide. Le résidu est chromatographié sur silice en éluant par un mélange toluène-acétate d'éthyle (95/5 ; v/v). On obtient 1,8 g du composé attendu sous forme d'une huile jaune clair.
RMN : 1,60-1,95 ppm : m : 4H ; 2,65-2,80 ppm : m : 2H ; 2,90-3,10 ppm : m : 2H ; 7,15-7,50 ppm : m : 2H ; 7,65 ppm : d : 1H.

### E) Acide 8-chloro-1-(2,4-dichlorophényl)-1,4,5,6-tétrahydro-1,2-diazabenzo[e] azulène-3-carboxylique.

Ce composé est préparé en procédant selon les étapes décrites à la préparation 6.
RMN : 2,00-2,30 ppm : m : 2H ; 2,55-3,25 : m : 4H ; 6,70 ppm d : 1H ; 7,20 ppm : d : 1H ; 7,50-7,80 ppm : m : 4H ; 13,1 : s : 1H.

En procédant comme décrit à la Préparation 1 ci-dessus, on obtient les composés suivants :

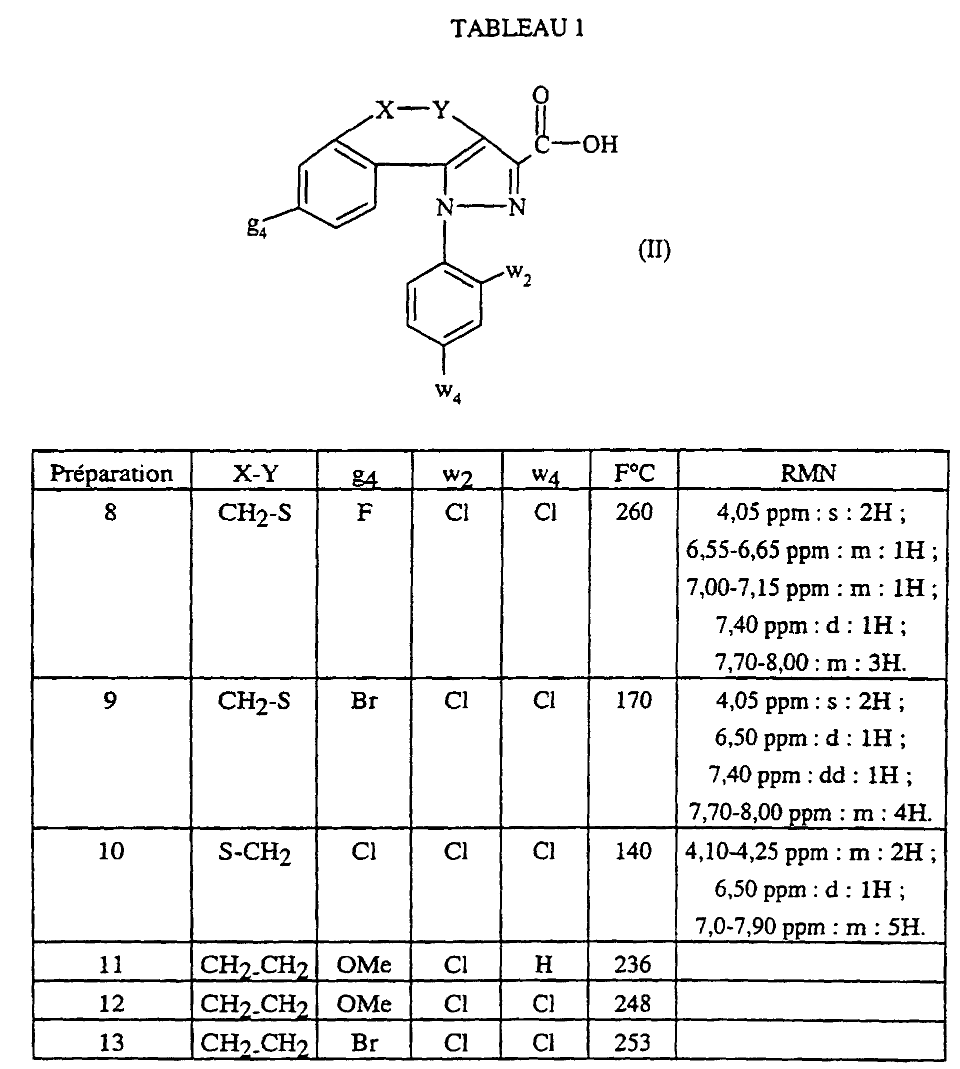

### EXEMPLE 1

### N-(pipérid-1-yl)-7-chloro-1-(2,4-dichlorophényl)-1,5-dihydro-isothiochroméno[4,3-c]pyrazole-3-carboxamide, hémihydrate.

I : g₄ = w₂ = w₄ = Cl, X-Y = CH₂-S,

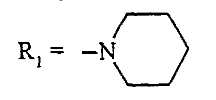

### A) Chlorure de l'acide 7-chloro-1-(2,4-dichlorophényl)-1,5-dihydro-isothiochroméno[4,3-c]pyrazole-3-carboxylique.

A un mélange de 0,7 g du composé obtenu à la Préparation 1 et 20 ml de toluène, on ajoute à TA 0,37 ml de chlorure de thionyle puis chauffe au reflux pendant 3 heures. Après refroidissement à TA, on concentre sous vide le mélange réactionnel, reprend le résidu avec 20 ml de toluène et évapore sous vide le solvant. On obtient 0,73 g du produit attendu sous forme d'huile que l'on utilise tel quel.

### B) N-(pipérid-1-yl)-7-chloro-1-(2,4-dichlorophényl)-1,5-dihydro-isothiochroméno[4,3-c]pyrazole-3-carboxamide, hémihydrate.

On refroidit à 0°C une solution de 0,2 ml de 1-aminopipéridine et 0,25 ml de triéthylamine dans 15 ml de DCM, ajoute goutte à goutte une solution de 0,73 g du composé obtenu à l'étape précédente dans 15 ml de DCM et laisse 18 heures sous agitation à TA. On verse le mélange réactionnel sur 200 ml d'eau, extrait au DCM, lave la phase organique à l'eau, par une solution saturée de NaCl, sèche sur Na₂SO₄ et évapore sous vide le solvant. On chromatographie le résidu sur gel de silice en éluant par le mélange toluène/AcOEt de (95/5 ; v/v) à (90/10 ; v/v). On obtient 0,32 g du composé attendu après cristallisation dans le mélange DCM/éther iso, F = 148°C.
RMN : 1,2-1,8 ppm : m : 6H ; 2,8 ppm : t : 4H ; 4,1 ppm : s : 2H ;
6,5-8,1 ppm : m : 6H ;
9,4 ppm : s : 1H.

### EXEMPLE 2

### N-(8-azaspiro[4.5]dec-8-yl)-7-chloro-1-(2,4-dichlorophényl)-1,5-dihydro-isothiochroméno[4,3-c] pyrazole-3-carboxamide.

I: g₄ = w₂ = w₄ = Cl, X-Y = CH₂-S,

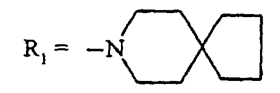

On refroidit à 0°C une solution de 0,28 g de chlorhydrate de 8-azaspiro[4.5]dec-8-ylamine et 0,41 ml de triéthylamine dans 15 ml de DCM, ajoute goutte à goutte 0,6 g du chlorure d'acide préparé à l'Exemple 1, étape A, dans 10 ml de DCM et laisse 4 heures sous agitation à TA. On extrait au DCM, lave la phase organique à l'eau, par une solution saturée de NaCl, sèche sur Na₂SO₄ et évapore sous vide le solvant. Le résidu est chromatographié sur gel de silice en éluant par le mélange toluène/AcOEt (95/5 ; v/v). On obtient 0,45 g du composé attendu sous forme amorphe.
RMN : 1,3-1,8 ppm : m : 12H ; 2,85 ppm : t : 4H ; 4,05 ppm : s : 2H ;
6,5-8,05 ppm : m : 6H ; 9,4 ppm : s : 1H.

On procède ensuite comme décrit dans les EXEMPLES ci-dessus pour préparer les composés selon l'invention rapportés dans le TABLEAU 2.

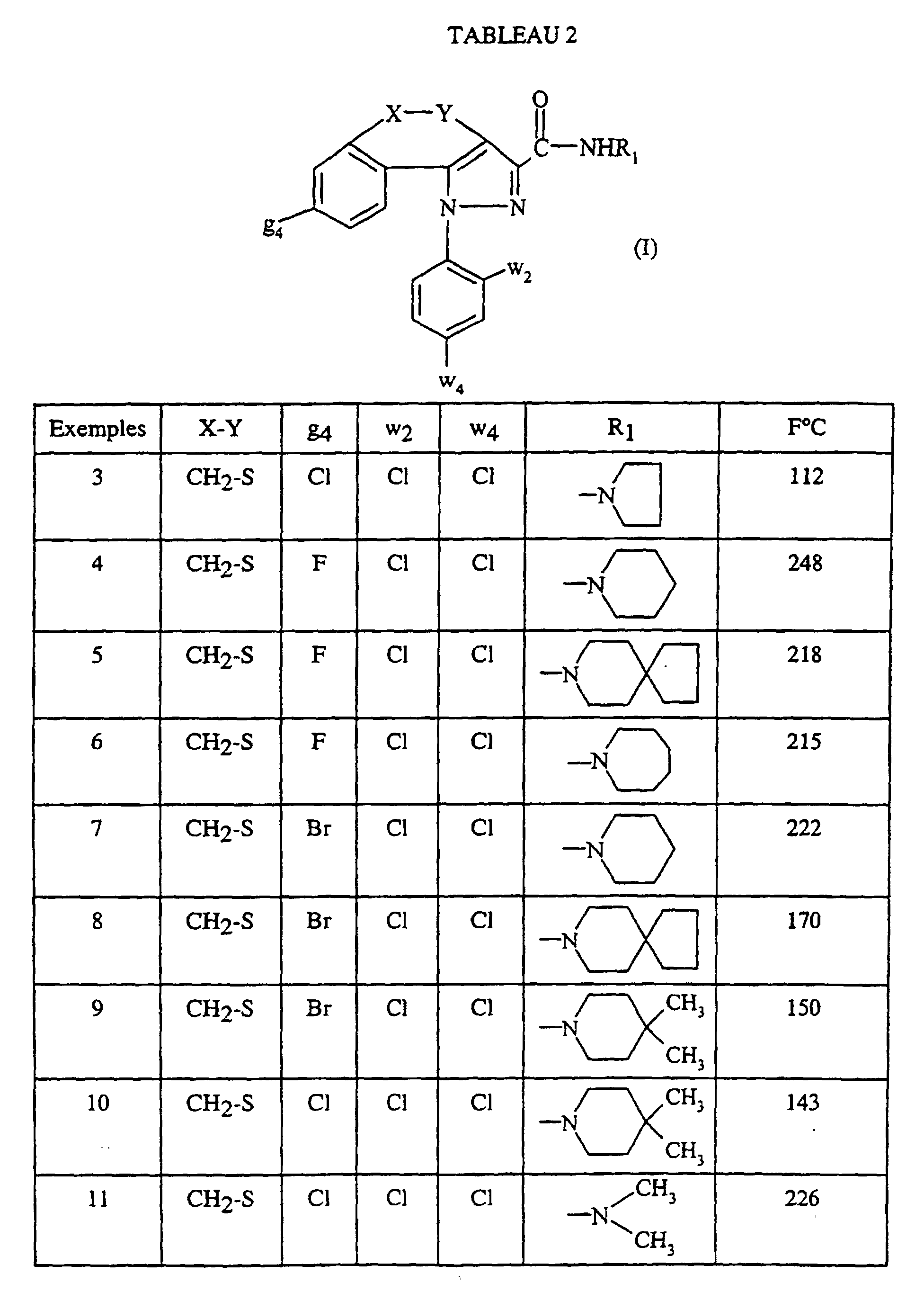

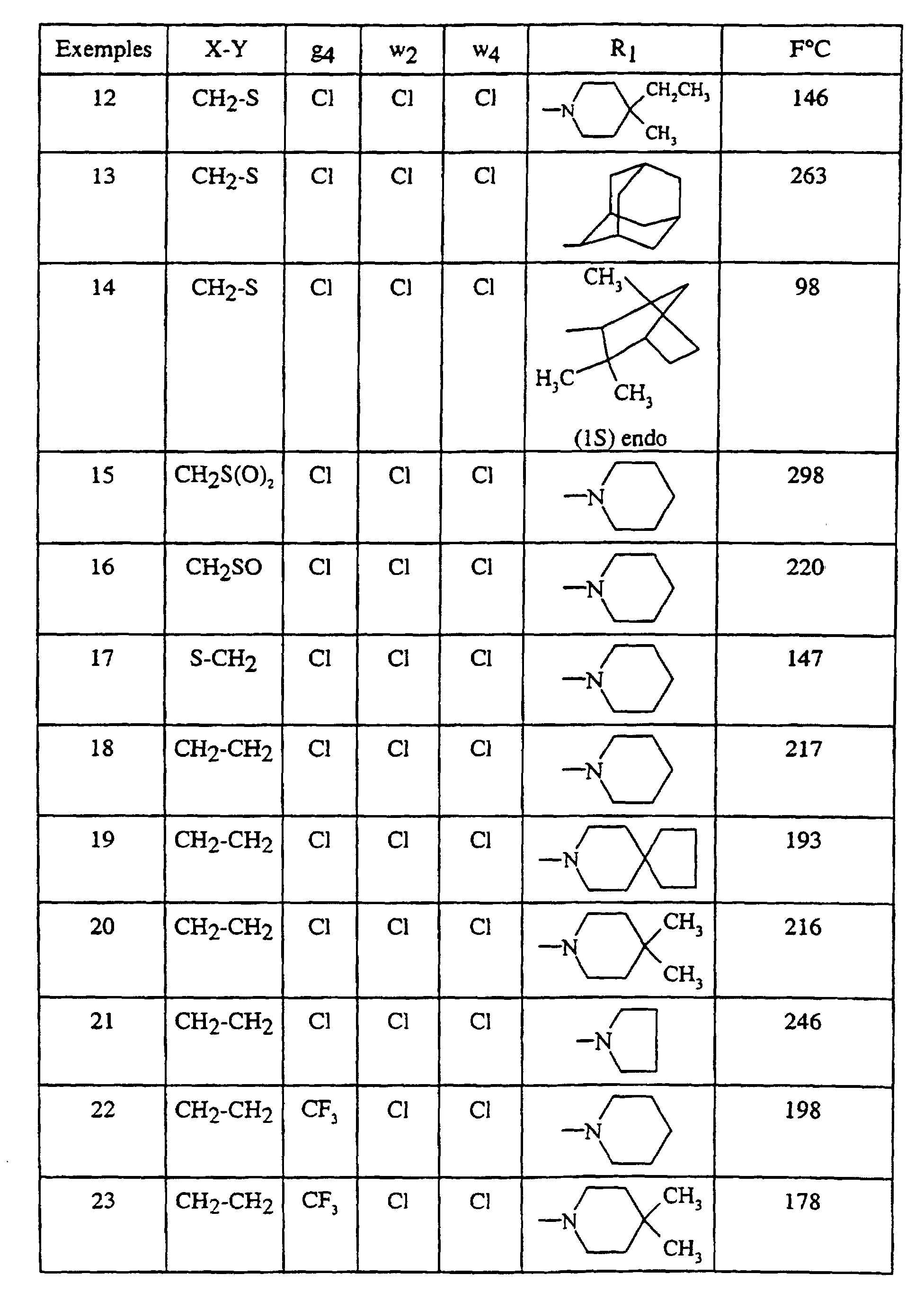

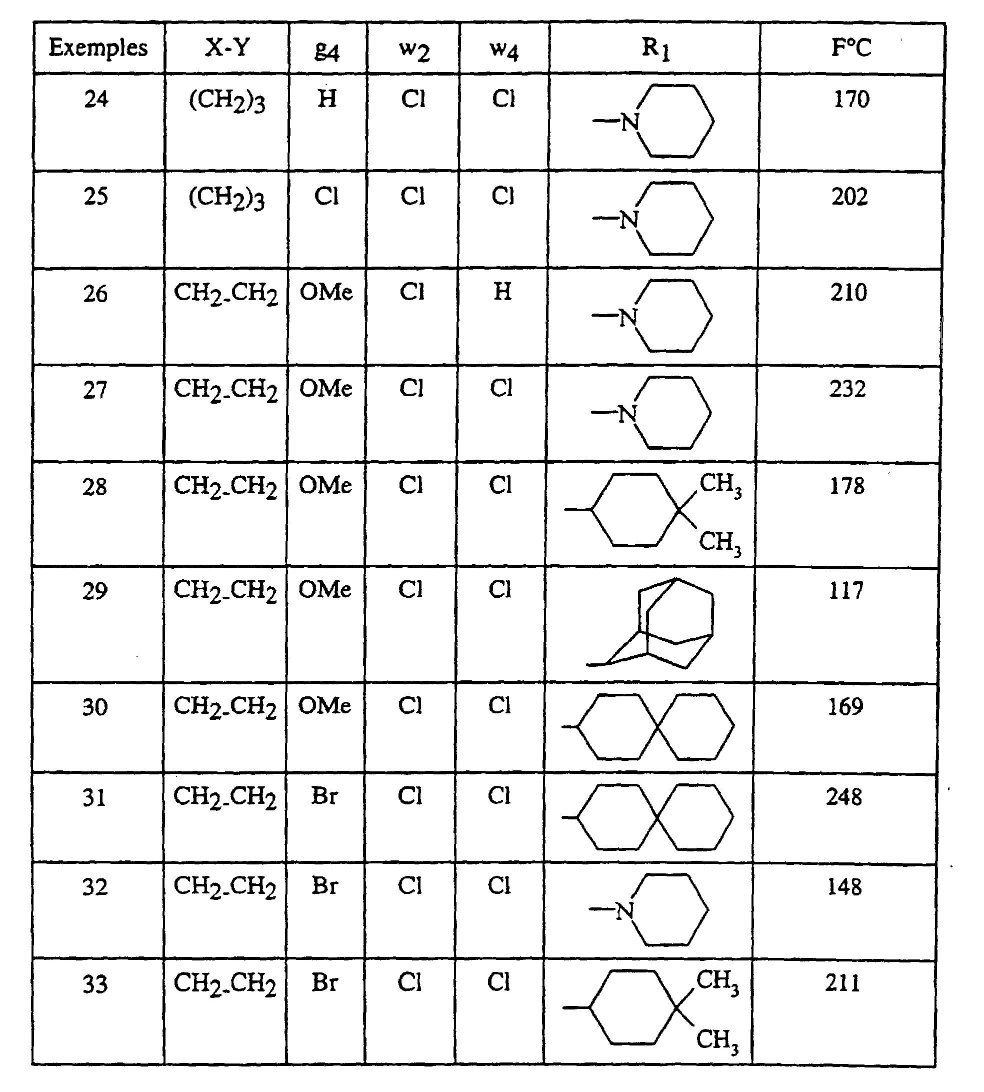

## Revendications

1. Un composé de formule : 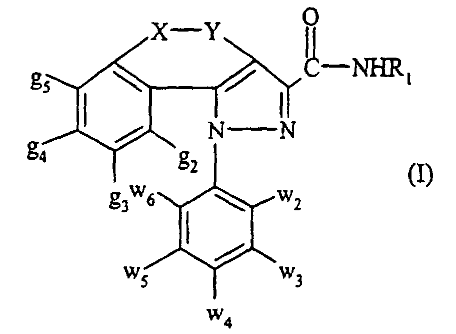 dans laquelle :
- X-Y- représente un groupe choisi parmi -(CH₂)ₙ-CH₂-, -CH₂-S(O)ₚ-, ou -S(O)ₚ-CH₂- ;
- n est égal à 1 ou 2 ;
- p est égal à zéro, 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₄)alkyle, un (C₁-C₄)alcoxy, un (C₁-C₄)alkylthio, un nitro ; au moins l'un des substituants w₂, w₃, w₄, w₅, w₆ étant différent de l'hydrogène ;
- R₁ représente un radical carbocyclique non aromatique en C₃-C₁₅ non substitué ou substitué une ou plusieurs fois par un (C₁-C₄)alkyle, ou un groupe NR₂R₃ ;
- R₂ et R₃ représentent chacun séparément l'hydrogène ou un (C₁-C₆)alkyle, ou R₂ et R₃ ensemble avec l'atome d'azote auquel ils sont liés constituent un radical hétérocyclique saturé ou insaturé de 5 à 10 chaînons, non substitué ou substitué une ou plusieurs fois par un (C₁-C₄)alkyle ;
ainsi que ses sels et leurs solvats.

2. Un composé selon la revendication 1 dans lequel g₂, g₃, g₅, w₃, w₅, w₆ représentent l'hydrogène et g₄, w₂ et w₄ ont l'une des valeurs définies ci-dessus pour les composés de formule (I) hormis l'hydrogène.

3. Un composé selon la revendication 2 dans lequel w₂ et w₄ représentent le chlore et g₄ représente le chlore ou le brome.

4. Un composé selon l'une quelconque des revendications 1 à 3 dans lequel R₁ représente un radical carbocyclique non aromatique en C₃-C₁₅ non substitué ou substitué une ou plusieurs fois par un (C₁-C₄)alkyle.

5. Un composé selon l'une quelconque des revendications1 à 3 dans lequel R₁ représente NR₂R₃, R₂ et R₃ constituant avec l'atome d'azote auquel ils sont liés un radical hétérocyclique saturé de 5 à 10 chaînons, non substitué ou substitué une ou plusieurs fois par un (C₁-C₄)alkyle.

6. Un composé selon l'une quelconque des revendications 1 à 5 dans lequel X-Y représente un groupe -(CH₂)ₙ-CH₂- avec n = 1 ou 2.

7. Un composé selon l'une quelconque des revendications 1 à 5 dans lequel X-Y représente -CH₂-S-.

8. Un procédé de préparation d'un composé selon la revendication 1, de ses sels et de ses solvats, **caractérisé en ce que** l'on traite un dérivé fonctionnel d'un acide de formule : 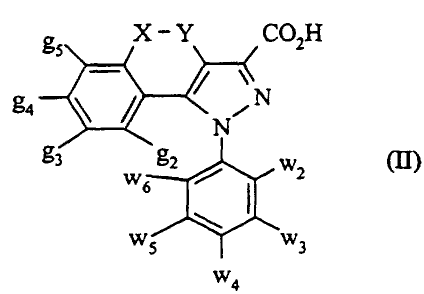 dans laquelle -X-Y- et g₂, g₃, g₄, g₅, w₂, w₃, w₄, w₅, w₆ sont tels que définis à la revendication 1 pour (I), avec un composé de formule NH₂R₁ (III), dans laquelle R₁ est tel que défini pour (I) à la revendication 1.

9. Un acide de formule : 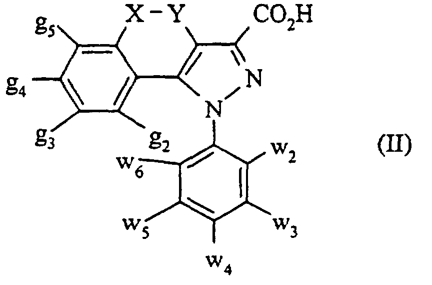 dans laquelle g₂, g₃, g₄, g₅, w₂, w₃, w₄, w₅, w₆ sont tels que définis pour (I) à la revendication 1 et ses dérivés fonctionnels, à la condition que :
- lorsque X-Y est -S-CH₂ ou -SO₂-CH₂-, et w₅ représente un fluor ou un trifluorométhyle, g₂, g₃, g₄, g₅, w₂, w₃, w₄, w₅, w₆ ne représentent pas simultanément l'hydrogène ;
- lorsque X-Y est -CH₂-CH₂-, w₄ représente un chlore, un fluor ou un méthoxy et g₄ représente l'hydrogène, un chlore ou un méthoxy, g₂, g₃, g₅, w₂, w₃, w₅, w₆ ne représentent pas simultanément l'hydrogène.

10. Un composé selon la revendication 9 dans lequel les dérivés fonctionnels sont choisis parmi le chlorure d'acide, l'anhydride, un anhydride mixte, un ester alkylique en C₁-C₄ dans lequel l'alkyle est droit ou ramifié, un ester activé par l'ester de *p*-nitrophényle, ou l'acide libre activé avec le N,N-dicyclohexylcarbodiimide ou avec l'hexafluorophosphate de benzotriazol-N-oxotris(diméthylamino)phosphonium.

11. Un composé selon la revendication 10 qui est l'ester alkylique en C₁-C₄ ou le chlorure d'acide.

12. Composition pharmaceutique contenant un composé selon l'une des revendications 1 à 7.

13. Composition pharmaceutique selon la revendication 12 sous forme d'unité de dosage.

14. Utilisation d'un composé selon la revendication 1 pour la préparation de médicaments destinés à traiter les maladies impliquant les récepteurs aux cannabinoïdes CB₁.

15. Utilisation selon la revendication 14, pour le traitement des troubles psychotiques et de la schizophrenie.

16. Utilisation selon la revendication 14, pour le traitement des troubles de l'appétit et de l'obésité,

17. Utilisation selon la revendication 14, pour le traitement des troubles mnésiques et cognitifs.

## Patentansprüche

1. Verbindung der Formel: 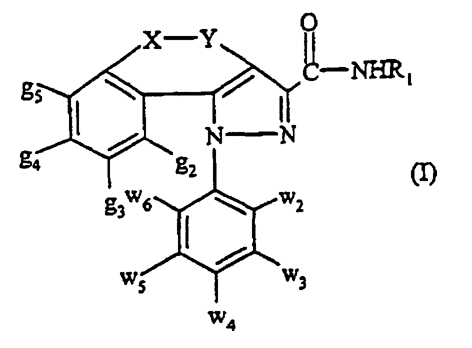 in der:
- X-Y- eine Gruppe bedeutet ausgewählt aus -(CH₂)ₙ-CH₂-, -CH₂-S(O)ₚ-, oder - S(O)ₚ-CH₂-;
- n 1 oder 2 bedeutet;
- p null, 1 oder 2 bedeutet;
- g₂, g₃, g₄, g₅, w₂, w₃, w₄, w₅ und w₆, die gleichartig oder verschieden sind, jeweils unabhängig voneinander Wasserstoff, Halogen, Trifluormethyl, (C₁-C₄)-Alkyl, (C₁-C₄)-Alkoxy, (C₁-C₄)-Alkylthio, Nitro bedeuten, wobei mindestens einer der Substituenten w₂, w₃, w₄, w₅ und w₆ von Wasserstoff verschieden ist;
- R₁ eine nicht-aromatische carbocyclische C₃-C₁₅-Gruppe, die nicht substituiert oder ein- oder mehrfach durch (C₁-C₄)-Alkyl substituiert ist, oder eine Gruppe NR₂R₃ bedeutet;
- R₂ und R₃ jeweils getrennt Wasserstoff oder (C₁-C₆)-Alkyl bedeuten oder R₂ und R₃ zusammen mit dem Stickstoffatom, an das sie gebunden sind, einen gesättigten oder ungesättigten heterocyclischen Rest mit 5 bis 10 Kettengliedern, der nicht substituiert oder ein- oder mehrfach durch (C₁-C₄)-Alkyl substituiert ist, bedeuten;
sowie deren Salze und deren Solvate.

2. Verbindung nach Anspruch 1, worin g₂, g₃, g₅, w₃, w₅ und w₆ Wasserstoff bedeuten und g₄, w₂ und w₄ eine der oben für die Verbindungen der Formel (I) angegebenen Bedeutungen mit Ausnahme von Wasserstoff besitzen.

3. Verbindung nach Anspruch 2, worin w₂ und w₄ Chlor und g₄ Chlor oder Brom bedeuten.

4. Verbindung nach einem der Ansprüche 1 bis 3, worin R₁ eine nicht-aromatische carbocyclische C₃-C₁₅-Gruppe, die nicht substituiert oder ein- oder mehrfach durch (C₁-C₄)-Alkyl substituiert ist, bedeutet.

5. Verbindung nach einem der Ansprüche 1 bis 3, worin R₁ NR₂R₃ darstellt, wobei R₂ und R₃ zusammen mit dem Stickstoffatom, in das sie gebunden sind, einen gesättigten heterocyclischen Rest mit 5 bis 10 Kettengliedern bilden, der nicht substituiert oder ein- oder mehrfach durch (C₁-C₄)-Alkyl substituiert ist.

6. Verbindung nach einem der Ansprüche 1 bis 5, worin X-Y eine Gruppe -(CH₂)ₙ-CH₂- darstellt, worin n 1 oder 2 bedeutet.

7. Verbindung nach einem der Ansprüche 1 bis 5, worin X-Y die Gruppe der Formel -CH₂-S- bedeutet.

8. Verfahren zur Herstellung einer Verbindung nach Anspruch 1, von ihren Salzen und ihren Solvaten, **dadurch gekennzeichnet, daß** man ein funktionelles Derivat einer Säure der Formel: 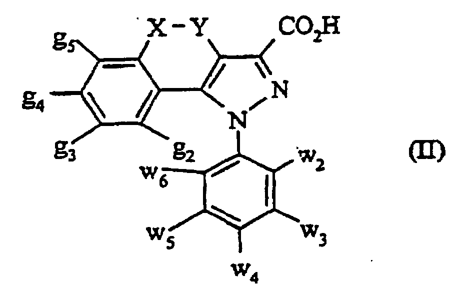 in der -X-Y- und g₂, g₃, g₄, g₅, w₂, w₃, w₄, w₅ und w₆ die in Anspruch 1 für (I) angegebenen Bedeutungen besitzen, mit einer Verbindung der Formel NH₂R₁ (III), in der R₁ die für (I) in Anspruch 1 angegebenen Bedeutungen besitzt, behandelt.

9. Säure der Formel: 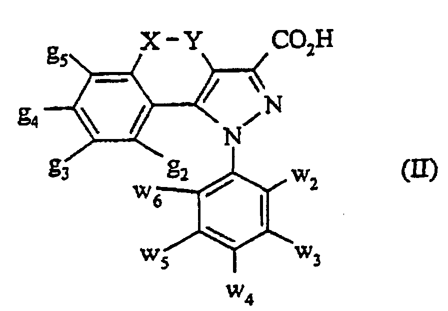 in der g₂, g₃, g₄, g₅, w₂, w₃, w₄, w₅ und w₆ die für (I) in Anspruch 1 angegebenen Bedeutungen besitzen und seine funktionellen Derivate, mit der Maßgabe, daß
- wenn X-Y -S-CH₂ oder -SO₂-CH₂- und w₅ ein Fluoratom oder eine Trifluormethyl-Gruppe bedeuten, g₂, g₃, g₄, g₅, w₂, w₃, w₄, w₅ und w₆ nicht gleichzeitig Wasserstoff bedeuten;
- wenn X-Y -CH₂-CH₂-, w₄ Chlor, Fluor oder Methoxy und g₄ Wasserstoff, Chlor oder Methoxy bedeuten, g₂, g₃, g₅, w₂, w₃, w₅ und w₆ nicht gleichzeitig Wasserstoff bedeuten.

10. Verbindung nach Anspruch 9, worin die funktionellen Derivate ausgewählt sind aus dem Säurechlorid, dem Anhydrid, einem gemischten Anhydrid, einem (C1-C4)-Alkylester, wobei der Alkylrest geradkettig oder verzweigt ist, einem durch den *p*-Nitrophenylester aktivierten Ester oder einer mit N,N-Dicyclohexylcarbodiimid oder mit Benzotriazol-N-oxotris(dimethylamino)-phosphonium-hexafluorphosphat aktivierten freien Säure.

11. Verbindung nach Anspruch 10, nämlich der (C₁-C₄)-Alkylester oder das Säurechlorid.

12. Pharmazeutische Zubereitung enthaltend eine Verbindung nach einem der Ansprüche 1 bis 7.

13. Pharmazeutische Zubereitung nach Anspruch 12 in Form einer Dosiseinheit.

14. Verwendung einer Verbindung nach Anspruch 1 für die Herstellung von Arzneimitteln zur Behandlung von Krankheiten, bei denen die Carrabinoid-Rezeptoren CB₁ beteiligt sind.

15. Verwendung nach Anspruch 14 für die Behandlung psychischen Störungen und der Schizophrenie.

16. Verwendung nach Anspruch 14 für die Behandlung von Appetitstörungen und der Fettsucht.

17. Verwendung nach Anspruch 14 für die Behandlung von Gedächtnis- und Erkenntnis-Störungen.

## Claims

1. Compound of formula: 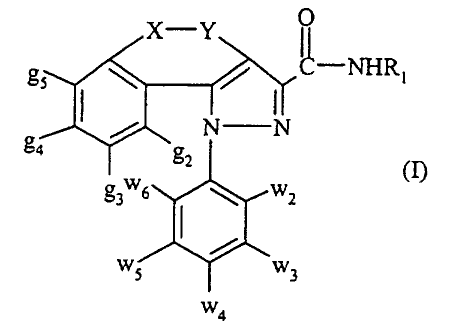 in which:
- X-Y- represents a group chosen from -(CH₂)ₙ-CH₂-,
- CH₂-S(O)ₚ- or -S(O)ₚ-CH₂-;
- n is equal to 1 or 2;
- p is equal to zero, 1 or 2;
- g₂, g₃, g₄, g₅, w₂, w₃, w₄, w₅, w₆ are identical or different and each independently represent hydrogen, a halogen, a trifluoromethyl, a (C₁-C₄)alkyl, a (C₁-C₄)alkoxy, a (C₁-C₄)alkylthio, a nitro; at least one of the substituents w₂, w₃, w₄, w₅, w₆ being different from hydrogen;
- R₁ represents a nonaromatic C₃-C₁₅ carbocyclic radical which is unsubstituted or substituted one or several times with a (C₁-C₄)alkyl, or an NR₂R₃ group;
- R₂ and R₃ each separately represent hydrogen or a (C₁-C₆)alkyl, or R₂ and R₃, together with the nitrogen atom to which they are attached, constitute a saturated or unsaturated 5- to 10-membered heterocyclic radical which is unsubstituted or substituted one or several times with a (C₁-C₄)alkyl;
as well as their salts and their solvates.

2. Compound according to Claim 1, in which g₂, g₃, g₅, w₃, w₅, w₆ represent hydrogen and g₄, w₂ and w₄ have one of the values defined above for the compounds of formula (I) except hydrogen.

3. Compound according to Claim 2, in which w₂ and w₄ represent chlorine and g₄ represents chlorine or bromine.

4. Compound according to any one of Claims 1 to 3, in which R₁ represents a nonaromatic C₃-C₁₅ carbocyclic radical which is unsubstituted or substituted one or several times with a (C₁-C₄)alkyl.

5. Compound according to any one of Claims 1 to 3, in which R₁ represents NR₂R₃, R₂ and R₃ constituting, with the nitrogen atom to which they are attached, a saturated 5- to 10-membered heterocyclic radical which is unsubstituted or substituted one or several times with a (C₁-C₄)alkyl.

6. Compound according to any one of Claims 1 to 5, in which X-Y represents a group -(CH₂)ₙ-CH₂- with n = 1 or 2.

7. Compound according to any one of Claims 1 to 5, in which X-Y represents -CH₂-S-.

8. Method for preparing a compound according to Claim 1, its salts and its solvates, **characterized in that** a functional derivative of an acid of formula: 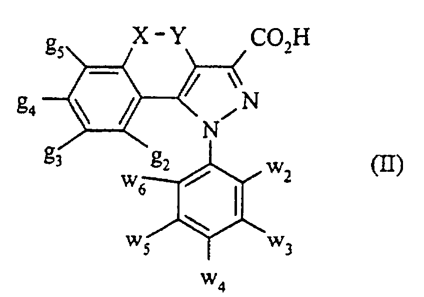 in which -X-Y- and g₂, g₃, g₄, g₅, w₂, w₃, w₄, w₅, w₆ are defined in Claim 1 for (I), is treated with a compound of formula NH₂R₁ (III) , in which R₁ is as defined for (I) in Claim 1.

9. Acid of formula: 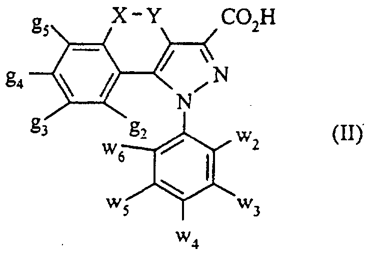 in which g₂, g₃, g₄, g₅, w₂, w₃, w₄, w₅, w₆ are as defined for (I) in Claim 1 and its functional derivatives, provided that:
- when X-Y is -S-CH₂ or -SO₂-CH₂-, and w₅ represents a fluorine or a trifluoromethyl, g₂, g₃, g₄, g₅, w₂, w₃, w₄, w₅, w₆ do not simultaneously represent hydrogen;
- when X-Y is -CH₂-CH₂-, w₄ represents a chlorine, a fluorine or a methoxy and g₄ represents hydrogen, a chlorine or a methoxy, g₂, g₃, g₅, w₂, w₃, w₅, w₆ do not simultaneously represent hydrogen.

10. Compound according to Claim 9, in which the functional derivatives are chosen from the acid chloride, the anhydride, a mixed anhydride, a C₁-C₄ alkyl ester in which the alkyl is straight or branched, an ester activated by *p*-nitrophenyl ester, or the free acid activated with N,N-dicyclohexylcarbodiimide or with benzotriazol-1-yloxytris(dimethylamino)phosphonium hexafluorophosphate.

11. Compound according to Claim 10, which is the C₁-C₄ alkyl ester or the acid chloride.

12. Pharmaceutical composition containing a compound according to one of Claims 1 to 7.

13. Pharmaceutical composition according to Claim 12, in the form of a dosage unit.

14. Use of a compound according to Claim 1 for the preparation of medicaments intended for treating diseases involving cannabinoid CB₁ receptors.

15. Use according to Claim 14, for the treatment of psychotic disorders and of schizophrenia.

16. Use according to Claim 14, for the treatment of appetite disorders and of obesity.

17. Use according to Claim 14, for the treatment of memory and cognitive disorders.
